# EUROPÄISCHE PATENTSCHRIFT

(11) **EP 0 634 393 B1**
(45) Veröffentlichungstag und Bekanntmachung des Hinweises auf die Patenterteilung: **17.09.1997**
(21) Anmeldenummer: 94250166.9
(22) Anmeldetag: 24.06.1994
(51) Int. Cl.: C07C 229/30, C08F 20/36, C08F 246/00, A61K 6/083

(54) **Polymerisierbare Enamine**
Polymerisable enamines
Enamines polymérisable

(30) Priorität: 12.07.1993 DE 4323617
(43) Veröffentlichungstag der Anmeldung: 18.01.1995
(73) Patentinhaber: IVOCLAR AG, FL-9494 Schaan (LI)
(72) Erfinder: Rheinberger, Volker, Dr., FL-9490 Vaduz (LI); Moszner, Norbert, Prof. Dr., FL-9492 Eschen (LI); Salz, Ulrich, Dr., D-88138 Weissensberg (DE)
(74) Vertreter: UEXKÜLL & STOLBERG

(56) Entgegenhaltungen:
- EP-A- 0 316 706
- EP-A- 0 492 847
- POLYM. BULL. (BERLIN) (POBUDR,01700839);94; VOL.32 (4); PP.419-26, IVOCLAR AG;SCHAAN; FL-9494; LIECHTENSTEIN (LI) Moszner N et al 'Reaction behavior of monomeric.beta.-ketoesters. 2. Synthesis, characterization and polymerization of methacrylate group containing enamines'
- Morrison,Boyd:Lehrbuch der organischen Chemie,1322 ff.(1986).
- Rauch-Puntingan,Völker:Acryl-und Methacrylverbindungen,85 ff.(1967).
- March : Advanced Organic Chemistry, 4th Ed. 896 ff. (1992)
- Methoden der Organischen Chemie (Houben-Weyl) Band VII/2b : Ketone, Teil II, Seiten 1944-5, 1948-51 (1976)

## Beschreibung

Die vorliegende Erfindung betrifft neue, polymerisierbare Verbindungen, die im Molekül neben einer Enamin-Funktion mindestens eine polymerisierbare ethylenische Gruppe enthalten.

Photopolymerisierbare Verbindungen bzw. Gemische, die Acryl-und/oder Methacrylsäureester enthalten, sind im Stand der Technik bekannt. In der DE-OS 37 38 864 werden Verbindungen beschrieben, die neben der polymerisierbaren Acryl- oder Alkacrylester-Funktion eine spezielle Aminogruppe enthalten.

Aufgabe der Erfindung war es, neue, sehr leicht zugängliche, polymerisierbare Verbindungen zur Verfügung zu stellen, die neben einer Enaminfunktion mindestens eine polymerisierbare ethylenische Gruppe enthalten und sich dadurch auszeichnen, daß sie zu einer neuen Klasse von Monomeren, Präpolymeren bzw. Polymeren führen, die sich durch geringe Variationen beim erfindungsgemäßen Herstellungsverfahren funktionalisieren bzw. hinsichtlich ihrer Eigenschaften auf den gewünschten Verwendungsbereich abstimmen lassen. Solche Enamine sollen unter anderem in Komposit-Materialien zur Herstellung von Werkstoffen mit elastomeren Eigenschaften eingesetzt werden können, wie zum Beispiel von Abformmaterialien oder temporären Füllungsmaterialien im Dentalbereich. Weiterhin soll diese neue Klasse von Verbindungen die Herstellung fester Kunststoffmatrizes und die einfache Synthese von Homo- und Copolymeren für unterschiedlichste Anwendungszwecke gestatten.

Zur Lösung der Aufgabe werden polymerisierbare Verbindungen der allgemeinen Formel (I) 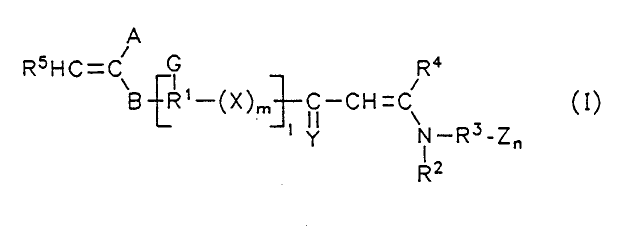 vorgeschlagen, wobei
l eine ganze Zahl ≥ 1 ist,
m und n jeweils 0 oder 1 sind,
   und
A Wasserstoff oder Alkyl,
B COO, CONH, C₆H₄ oder Arylen,
R¹ Alkylen, Oxyalkylen, C₆H₄ oder Arylen,
R² Wasserstoff, Alkyl oder Aryl
R³ Wasserstoff, Alkyl oder Aryl bedeutet, wenn n = 0 ist,
R³ Alkylen oder Arylen bedeutet, wenn n = 1 ist,
   wobei
R² und R³ nicht gleichzeitig Wasserstoff bedeuten,
R⁴ Alkyl,
R⁵ Wasserstoff, C₆H₅, CO₂H, CO₂Alkyl oder CN
G Wasserstoff bedeutet,
X Sauerstoff, Schwefel oder Imino,
Y Sauerstoff oder Schwefel
   bedeuten und
Z für eine oder mehrere funktionelle Gruppen steht.

Die allgemeine Bezeichnung Alkyl und Alkylen kann für gesättigte und/oder ungesättigte Kohlenwasserstoff-Reste mit 1 bis 12 oder mehr als 12 Kohlenstoffatomen stehen, wobei die Alkyl- und Alkylen-Gruppen untereinander jeweils gleich oder verschieden sein können.

Die erfindungsgemäßen photopolymerisierbaren Verbindungen können auch mindestens zwei Struktureinheiten der allgemeinen Formel (I) enthalten, in welchen l, m, n, A, B, R¹, R³, R⁴, R⁵, X, Y und Z die oben angegebene Bedeutung haben, aber G und/oder R² die Struktureinheiten verknüpfende Brückensubstituenten sind.

Erfindungsgemäß wird ferner ein Verfahren zur Herstellung der photopolymerisierbaren Verbindungen vorgeschlagen, bei dem man die Verbindung der allgemeinen Formel (II) 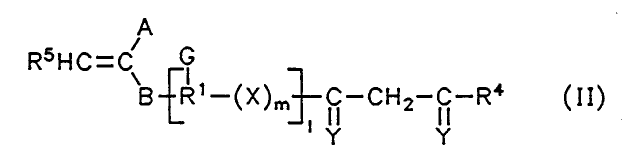 mit einem primären Amin der allgemeinen Formel NH₂R², mit einem sekundären Amin der allgemeinen Formel NHR²R³, mit einem funktionalisierten Amin der allgemeinen Formel NHR²R³-Z oder mit einem Diamin der allgemeinen Formel NHR³-R²-R³NH oder NH(R³Z)-R²-(R³Z)NH umsetzt, wobei l, m, n, A, B, R¹, R², R³, R⁴, R⁵, X, Y, Z und G die oben angegebene Bedeutung haben.

Die erfindungsgemäßen polymerisierbaren Verbindungen liegen entweder als Monomer oder als Präpolymer vor und können unter anderem zur Herstellung von Präpolymeren bzw. Polymeren sowie von Homo- oder Copolymeren mit funktioneller Enamin-Gruppe verwendet werden, insbesondere zur Herstellung elastomerer Werkstoffe und in besonders geeigneter Weise zur Herstellung von Dentalmaterialien.

Die vorliegende Erfindung basiert auf dem grundlegenden Konzept, Verbindungen der allgemeinen Formel (II) mit Aminen umzusetzen (vgl. Fig. 1), wobei als polymerisierbare ethylenische Gruppen unter anderem Acryl, Methacryl, Styryl usw. eingesetzt werden können und mit Ausnahme von tertiären Aminen primäre, sekundäre, und funktionalisierte Monoamine sowie allgemein Diamine in Betracht kommen. 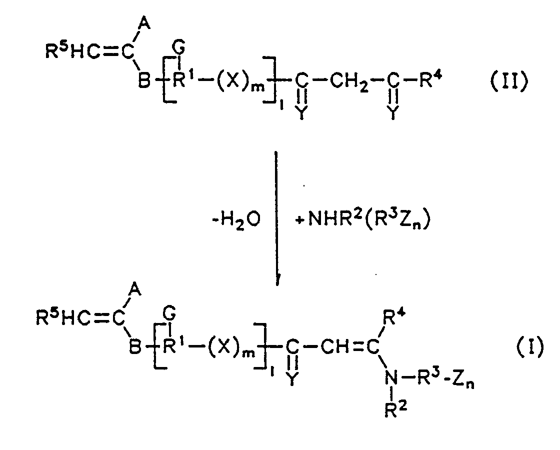
- *Fig. 1:*: *Grundlegendes Konzept zur Herstellung der erfindungs-* *gemäßen, polymerisierbare ethylenische Gruppen enthal-* *tenden Enamine der allgemeinen Formel (I).*

So führt die Umsetzung mit primären oder sekundären Aminen in hohen Ausbeuten zu speziellen Enaminen mit mindestens einer polymerisierbaren ethylenischen Gruppe. Vorzugsweise werden Methacrylat-Gruppen enthaltende β-Dicarbonylverbindungen eingesetzt, wie zum Beispiel 2-Acetoacetoxyethylmethacrylat (AAEMA), (Fig. 2). 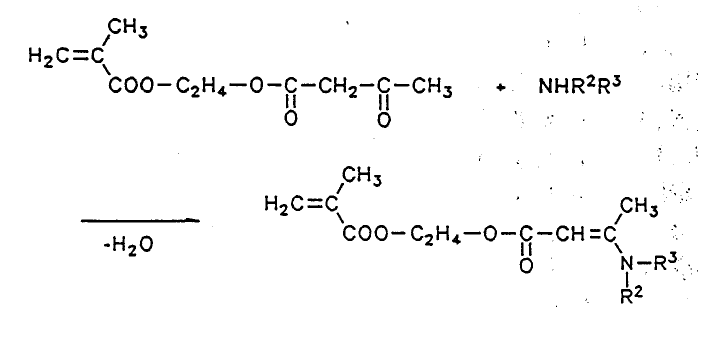
- *Fig. 2:*: *Umsetzung von AAEMA mit primären und sekundären Aminen.*

Auf diese Weise hergestellte Verbindungen sind in der Literatur noch nicht bekannt, und die Zahl der einzusetzenden Amine ist praktisch unbegrenzt. Neben beispielsweise Methacrylat-Gruppen enthaltenden β-Dicarbonyl- oder β-Dithiocarbonylverbindungen können außerdem andere Verbindungen mit Aminen umgesetzt werden, die polymerisierbare ethylenische Gruppen wie zum Beispiel Styryl, Acryl usw. enthalten.

Während die Umsetzungen solcher Carbonylverbindungen mit primärem Aminen relativ rasch und in hohen Ausbeuten verlaufen, müssen die Reaktionen mit sekundären oder auch aromatischen Aminen in der Regel katalysiert werden, liefern aber in diesen Fällen die erfindungsgemäßen Verbindungen der allgemeinen Formel (I) in ebenso hohen Ausbeuten.

Die auf dem in Fig. 2 dargestellten Weg erhaltenen, polymerisierbaren Enamine lassen sich bevorzugt als Monomere in Komposit-Materialien einsetzen, bei deren Polymerisation Werkstoffe mit äußerst vorteilhaften, elastomeren Eigenschaften mit variierbarer Shore-A-Härte bzw. Zug-Dehnungs-Charakteristik erhalten werden. Diese Polymere lassen sich vorzugsweise im Dentalbereich einsetzen, wie zum Beispiel für Abformmaterialien, Füllungsmaterialien, insbesondere temporäre Füllungsmaterialien und Unterfüllungsmaterialien sowie Unterfütterungsmaterialien.

Werden anstelle von primären oder sekundären Aminen entsprechende funktionalisierte Amine eingesetzt, so können auf diese Weise Monomere erhalten werden (Fig. 3), die nach den im Stand der Technik bekannten Methoden nur äußerst schwer zugänglich sind. So führt z.B. die Umsetzung von Aminoalkoholen mit (Meth)acrylsäurechlorid sowohl zu einer Acylierung der Amino- als auch der OH-Gruppe, während durch die Enaminbildung ein polymerisationsfähiges Derivat mit freier alkoholischer OH-Gruppe gebildet wird. 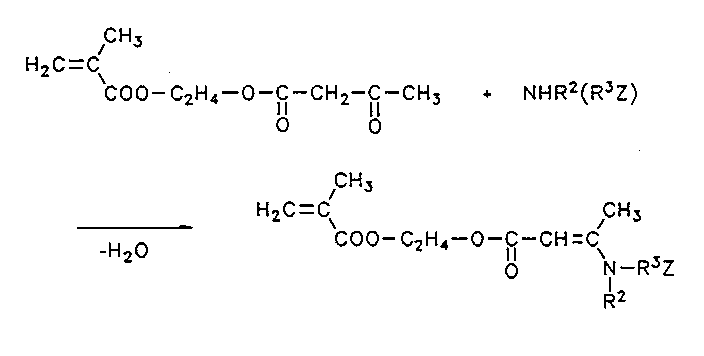
- *Fig. 3:*: *Umsetzung von AAEMA mit funktionalisierten Aminen.*

Bei Verwendung von Diaminen, wie z.B. Hexamethylendiamin, lassen sich auf einfachem Wege polymerisierbare Verbindungen herstellen, bei denen zwei Struktureinheiten der allgemeinen Formel (I) über einen Brückensubstituenten R² miteinander verknüpft sind (vgl. Fig. 4). 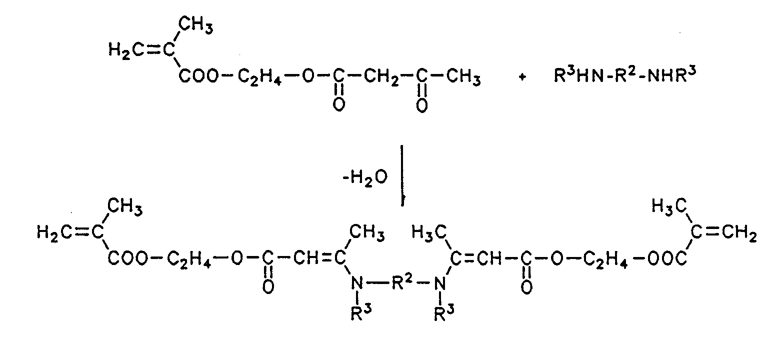
- *Fig. 4:*: *Umsetzung von AAEMA mit Diaminen.*

Zur Herstellung von polymerisierbaren Verbindungen, die die Struktureinheit (I) mehrfach enthalten, lassen sich auch β-Dicarbonyl- bzw. β-Dithiocarbonylverbindungen der allgemeinen Formel (II) einsetzen, die einen Brückensubstituenten G enthalten, der bereits vor der Reaktion mit dem Amin zwei Struktureinheiten (I) miteinander verknüpft. Durch die Umsetzung mit Diaminen erfolgt dann die weitere Verknüpfung über den Brückensubstituenten R², der über das Diamin eingeführt wird.

Beim Einsatz von Diaminen läßt sich auf diese Weise eine sogenannte Zwei-Phasen-Härtung realisieren, die in Fig. 5 beispielhaft dargestellt ist. Im ersten Schritt reagiert die polymerisierbare Verbindung, die zwei, über einen Brückensubstituenten G verknüpfte Struktureinheiten der allgemeinen Formel (I) enthält (in Fig. 5 z.B. das Umsetzungsprodukt von 2,2-Bis[4-(2-hydroxy-3-methacryloxypropyloxy)phenyl]propan (Bis-GMA) mit Diketen), mit einem Diamin unter Bildung eines über die Brückensubstituenten R² und G verknüpften Präpolymers. 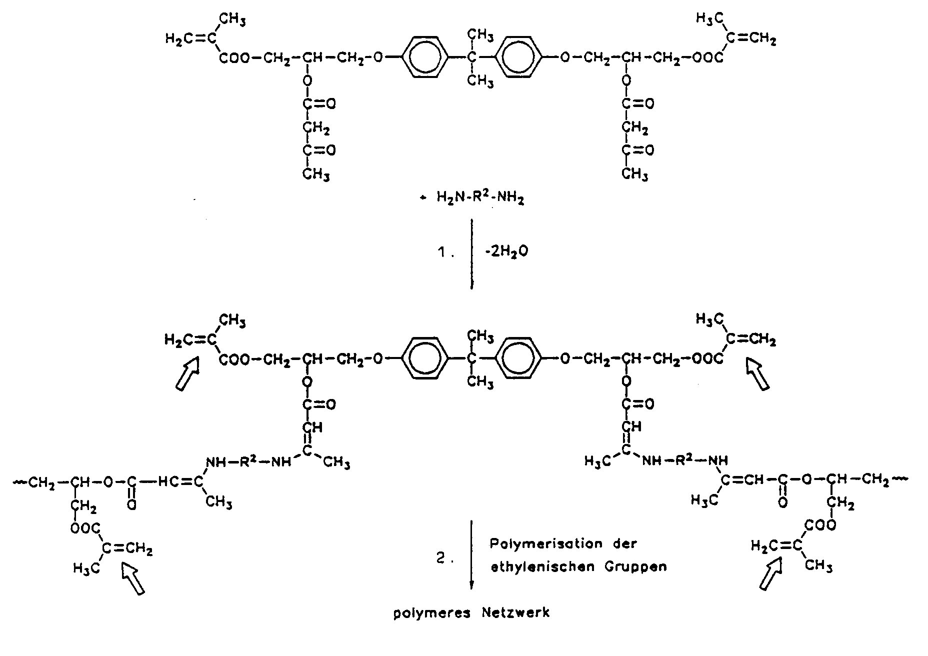
- *Fig. 5:*: *Beispielhafte Darstellung einer Zweiphasenhärtung.*

Im nächsten Schritt kann das elastische bzw. hochviskose Präpolymer sowohl durch Homo- als auch durch Copolymerisation über die vorhandenen ethylenischen Gruppen, beispielsweise durch radikalische Copolymerisation mit Triethylenglykoldimethacrylat (TGDMA), zu einer vernetzten, festen Kunststoffmatrix polymerisiert werden.

Die erfindungsgemäßen Verbindungen der allgemeinen Formel (I) sowie das erfindungsgemäße Verfahren zur Herstellung dieser Verbindungen sind weiterhin zur bequemen Funktionalisierung von Polymeren von äußerst großem Nutzen.

Im Stand der Technik werden zur Funktionalisierung von Polymeren häufig entsprechende funktionelle Seitengruppen über polymeranaloge Umsetzungen eingeführt. Diese polymeranalogen Reaktionen laufen aber meist nur unvollständig, d.h. mit geringen Ausbeuten ab.

Es ist bekannt, daß sich Enamine, die bei unterschiedlichen Reaktionstypen häufig als Zwischenstufe auftreten, durch eine hohe Reaktivität auszeichnen. Mit den erfindungsgemäßen Verbindungen der allgemeinen Formel (I) sind auf extrem einfache Weise Homo- oder Copolymere zugänglich, die derartige, hoch reaktive Enaminfunktionen aufweisen. Solche reaktiven Homo- oder Copolymere lassen sich im Gegensatz zu den meisten Polymeren im Stand der Technik mit sehr hohen Umsätzen, d.h. mit hohen Ausbeuten, polymeranalog funktionalisieren und stellen damit einen großen Fortschritt gegenüber den bislang bei der Funktionalisierung von Polymeren angewendeten Methoden dar (vgl. Fig. 6). 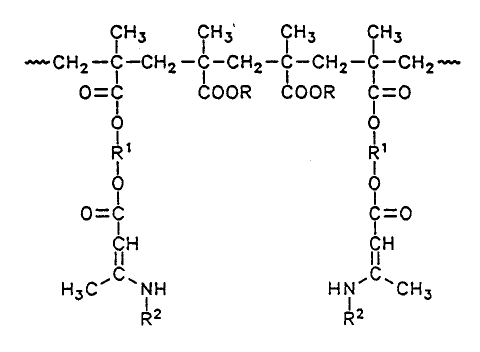
- *Fig. 6:*: *Copolymer der allgemeinen Formel (I), das sich aufgrund* *der vorhandenen Enaminfunktion für polymeranaloge Umsetzungen eignet.*

Wie bereits oben erwähnt ist, eignen sich die polymerisierbaren Verbindungen der allgemeinen Formel (I) in besonders geeigneter Weise zur Herstellung von Polymeren für unterschiedlichste Anwendungszwecke. Dies ist auf die Tatsache zurückzuführen, daß die die Struktureinheit (I) enthaltenden erfindungsgemäßen Verbindungen durch das gleichzeitige Vorliegen einer Enamin sowie einer polymerisierbaren ethylenischen Gruppe zu einer Vielzahl unterschiedlicher Reaktionen und Funktionalisierungen befähigt sind. Durch die Einführung von Brückensubstituenten G und/oder R², die zwei oder mehrere Struktureinheiten der allgemeinen Formel (I) miteinander verknüpfen, lassen sich die Einsatzmöglichkeiten noch weiter steigern. Mit Hilfe des erfindungsgemäßen Verfahrens zur Herstellung der beanspruchten Verbindungen der allgemeinen Formel (I), d.h. durch Umsetzung der β-Dicarbonyloder β-Dithiocarbonylverbindungen der allgemeinen Formel (II) mit Aminen, sind neue Klassen von Monomeren, Präpolymeren bzw. Polymeren zugänglich. Die erfindungsgemäßen Verbindungen sind im Stand der Technik nicht bekannt, und aufgrund der einfachen Variierbarkeit ihrer charakteristischen Eigenschaften sind sie für eine Vielzahl von Einsatzmöglichkeiten von größter Bedeutung. Die durch die vorliegende Erfindung zugänglichen elastomeren Werkstoffe haben sich insbesondere bei der Verwendung im Dentalbereich ausgezeichnet, zum Beispiel als Abformmaterialien, Füllungsmaterialien, vorzugsweise temporäre Füllungsmaterialien, Unterfüllungs- oder Unterfütterungsmaterialien.

Die vorliegende Erfindung wird im folgenden anhand von Beispielen näher erläutert.

### Beispiel 1

### Allgemeine Arbeitsvorschrift Umsetzung von β-Dicarbonyl- bzw. β-Dithiocarbonylverbindungen der allgemeinen Formel (II) mit Aminen:

50 mmol (II) wurden zunächst in 40 ml Tetrahydrofuran (THF) gelöst. Dazu ließ man innerhalb von 10 Minuten eine Lösung von 50 mmol Amin bzw. 25 mmol Diamin in 20 ml THF zutropfen und rührte für weitere 6 Stunden bei Raumtemperatur. Im Anschluß wurde die Reaktionslösung nach Zugabe von ca. 100 ml Ether zweimal mit je 20 ml 1,0 M Natronlauge und mit Wasser ausgeschüttelt. Die organische Phase wurde über wasserfreiem Natriumsulfat getrocknet. Nach Abziehen des Ethers im Wasserstrahlvakuum engte man im Feinvakuum bis zur Gewichtskonstanz ein. Bei Verwendung von Aminocarbonsäuren wurde Methanol als Lösungsmittel verwendet und nicht mit Natronlauge ausgeschüttelt. Die flüssigen Enamine wurden ohne weitere Reinigung charakterisiert, während die festen Produkte aus Wasser/Ethanol umkristallisiert wurden.

Zur Stabilisierung der Monomere wurden 100 ppm 2,6-Ditertiärbutylphenol (BHT) zugegeben.

### Beispiel 2

### Umsetzung von 2-Acetoacetoxyethylmethacrylat (AAEMA) mit primären bzw. funktionalisierten, primären Monoaminen:

Bei der Umsetzung von AAEMA mit verschiedenen primären bzw. funktionalisierten, primären Monoaminen gemäß Beispiel 1 wurden die N-monosubstituierten Methacryloyloxyethyl-3-aminoprotonate 1a-f erhalten (Tabelle 1).

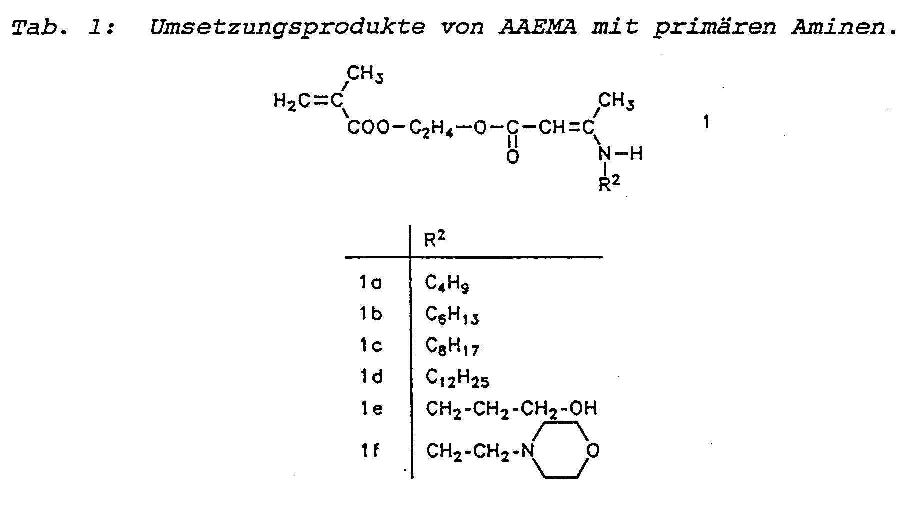

### Beispiel 3

Bei der Umsetzung von Polyethylenglykol-200-(2-Acetoacetoxymethacrylat) (PEG-200-AAMA) mit n-Butylamin gemäß Beispiel 1 wurde die in Fig. 7 gezeigte Verbindung erhalten. 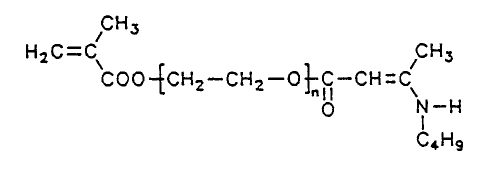
- *Fig. 7:*: *Umsetzungsprodukt von PEG-200-AAMA mit n-Butylamin.*

### Beispiel 4

Bei der Umsetzung von AAEMA mit Aminosäuren gemäß Beispiel 1 wurden die in Tabelle 2 dargestellten Verbindungen erhalten.

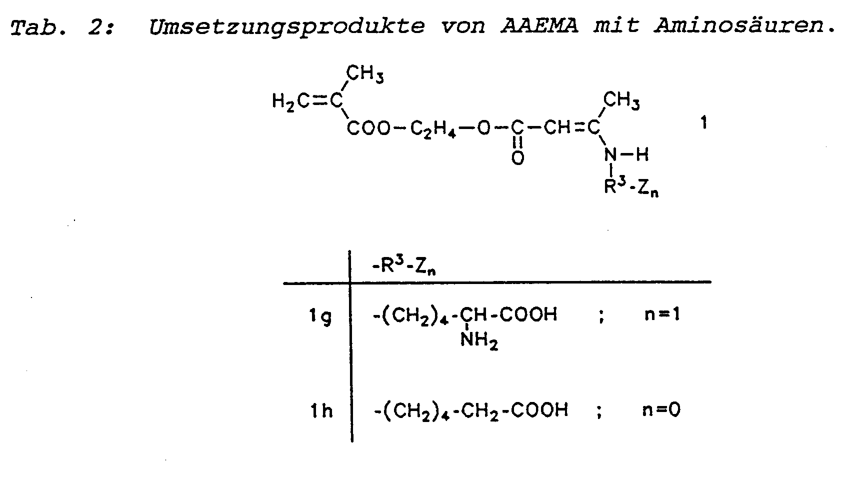

### Beispiel 5

### Umsetzung von AAEMA mit primären Diaminen:

Bei der Umsetzung von AAEMA mit verschiedenen Diaminen gemäß Beispiel 1 wurden die polymerisationsfähigen Dimethacryloyloxyethyl-(alkylenbis-3-N-aminocrotonate) 2a-f erhalten (Tabelle 3).

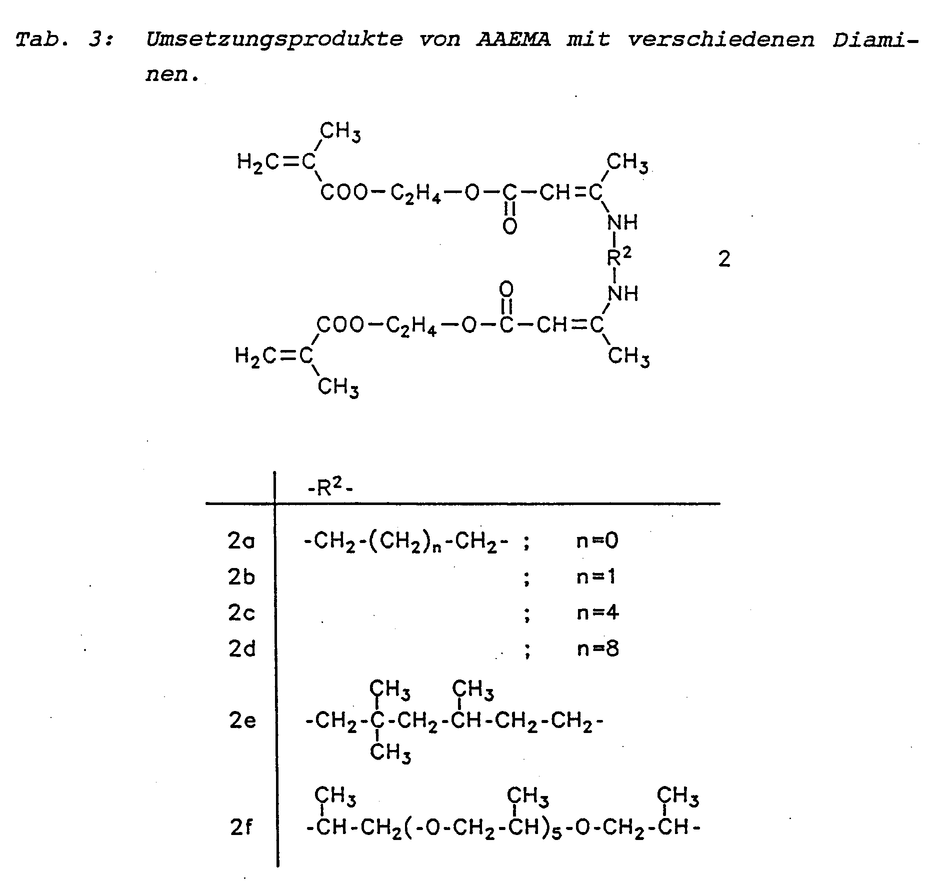

### Beispiel 6

### Charakterisierung der in den Beispielen 2 bis 5 erhaltenen Verbindungen:

1a: Viskose Flüssigkeit, Ausbeute 79,3 %; C₁₄H₂₃NO₄ (269,3 g/mol): gef.: C: 62,21 (ber.: 62,43), H: 8,52 (8,60), N: 5,13 (5,20); IR (Film): 3288 (NH), 1721 (C=O), 1654 (C=O), 1607 cm⁻¹ (C=C).
1b: Viskose Flüssigkeit, Ausbeute: 80,6 %; C₁₆H₂₇NO₄ (297,4 g/mol): C: 64,46 (64,62), H: 9,21 (9,15), N: 4,59 (4,71); IR (Film): 3289 (NH), 1721 (C=O), 1654 (C=O), 1606 cm⁻¹ (C=C).
1c: Viskose Flüssigkeit, Ausbeute: 85,4 %; C₁₈H₃₁NO₄ (325,5 g/mol): C: 66,22 (66,43), H: 9,54 (9,60), N: 4,29 (4,03); IR (Film): 3288 (NH), 1721 (C=O), 1654 (C=O), 1608 cm⁻¹ (C=C).
1d. Viskose Flüssigkeit, Ausbeute: 87,2 %; C₂₂H₃₉NO₄ (381,6 g/mol): C: 69,12 (69,25), H: 10,14 (10,30), N: 3,63 (3,67); IR (Film): 3284 (NH), 1722 (C=O), 1655 (C=O), 1608 cm⁻¹ (C=C).
1e: Viskose Flüssigkeit, Ausbeute: 74,4 %, IR (Film): 3391 (OH), 3311 (NH), 1720 (C=O), 1652 (C=O), 1605 cm⁻¹ (C=C).
1f: Viskose Flüssigkeit, Ausbeute: 81,0 %; IR (Film): 3287 (NH), 1719
1g: Viskose Flüssigkeit, Ausbeute: 57,8 %; IR (Film): 3395 (OH), 3288 (NH), 1720 (C=O), 1650 (C=O), 1601 cm⁻¹ (C=C).
1h: Sehr viskose Flüssigkeit, Ausbeute: 62,1 %,; IR (Film): 3400 (OH), 3289 (NH), 1718 (C=O), 1656 (C=O), 1633 (C=C), 1603 cm⁻¹ (C=C).
2a: Feststoff, Fp.: 112 °C, Ausbeute: 78,2 %; C₂₂H₃₂N₂O₈ (452,5 g/mol): C: 58,40 (58,40), H: 7,06 (7,13), N: 6,17 (6,19); IR (KBr): (NH), 1709 (C=O), 1675 (C=O), 1630 (C=C), 1592 cm⁻¹ (C=C)
2b: Feststoff, Fp.: 88°C, Ausbeute: 79,0 %; C₂₃H₃₄N₂O₈ (466,5 g/mol): C: 59,14 (59,21), H: 7,27 (7,35), N: 5,98 (6,02); IR (KBr): 3300 (NH), 1713 (C=O), 1667 (C=O), 1597 cm⁻¹ (C=C)).
2c: Feststoff, Fp.: 67 °C, Ausbeute: 83,7 %; C₂₆H₄₀N₂O₈ (508,6 g/mol): C: 61,33 (61,41), H: 7,91 (7,93), N: 5,44 (5,51); IR (KBr): 3300 (NH), 1713 (C=O), 1655 (C=O), 1639 (C=C), 1596 cm⁻¹ (C=C)).
2d: Feststoff, Fp.: 38 °C; Ausbeute: 81,3 %; C₃₀H₄₈N₂O₈ (564,7 g/mol): C: 63,66 (63,81), H: 8,42 (8,57), N: 5,06 (4,96); IR (KBr): 3284 (NH), 1722 (C=O), 1653 (C=O), 1603 cm⁻¹ (C=C).
2e: hochviskose Flüssigkeit, Ausbeute: 86,3%; IR (Film): 3280 (NH), 1720 (C=O), 1651 (C=O), 1610 cm⁻¹ (C=C).
2f: viskose Flüssigkeit, Ausbeute: 78,9 %; IR (Film): 3284 (NH), 1721 (C=O), 1655 (C=O), 1606 cm⁻¹ (C=C).
3: hochviskose Flüssigkeit; Ausbeute 74,6%; IR (Film): 3284 (NH), 1719 (C=O), 1651 (C=O), 1606 cm⁻¹ (C=C).
AAEMA: IR (Film): 1740 (C=O), 1720 (C=O,), 1637 cm⁻¹ (C=C).

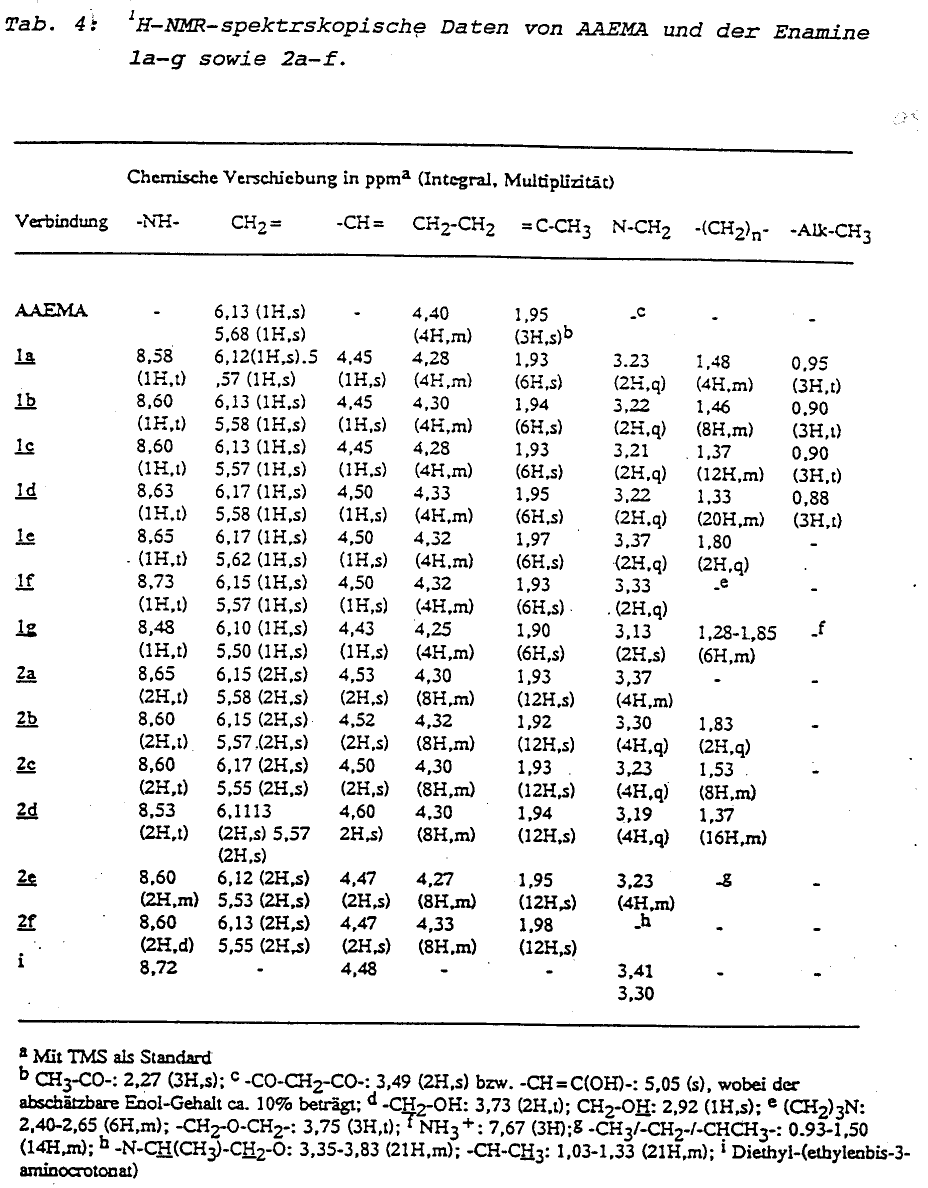

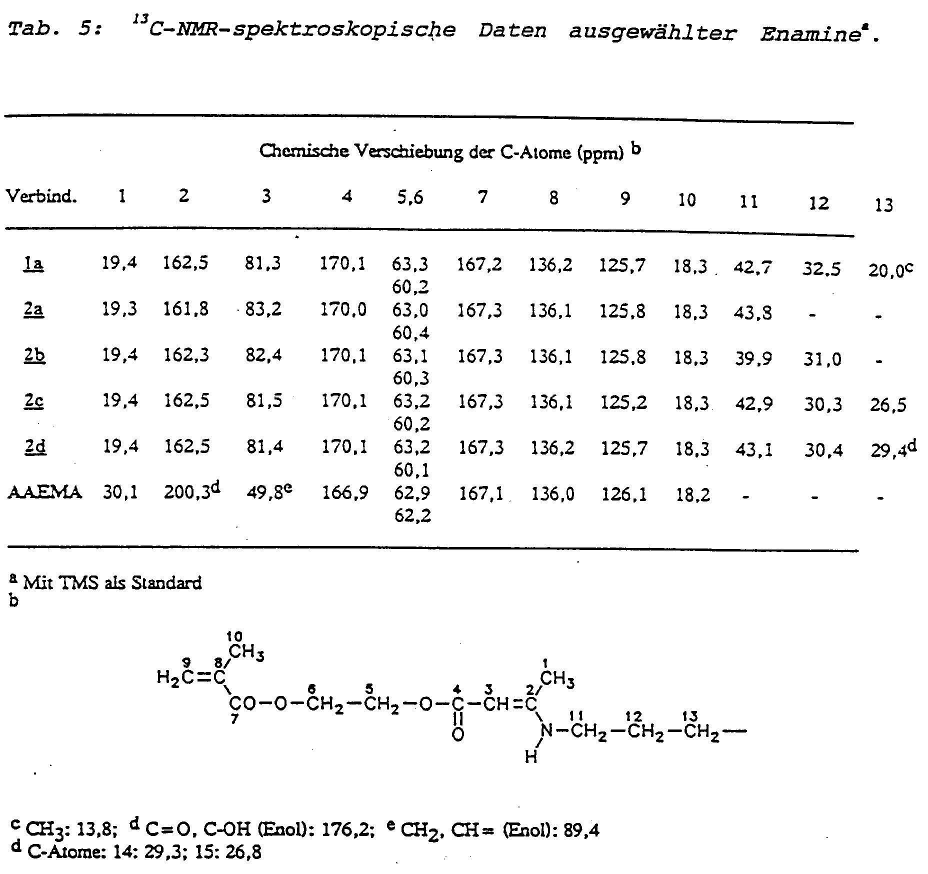

### Beispiel 7

### Verwendung der erfindungsgemäßen Verbindungen zur Herstellung von Dentalwerkstoffen:

1) Feste Füllungsmaterialien;
   (a) 31,7 Gewichtsteile des Enamins aus AAEMA und 2,2,4-Trimethylhexamethylendiamin (Monomer 2e)
   (b) 7,8 Gewichtsteile Decandioldimethacrylat,
   (c) 41,3 Gewichtsteile silanisierte pyrogene Kieselsäure (AEROSIL® Ox-50 der Degussa AG) und
   (d) 18,6 Gewichtsteile röntgenopakes YbF3
   vermischte man mit 0,3 Gewichtsteilen Campherchinon (CC), 0,5 Gewichtsteilen N,N-(2-Cyanoethyl)methylanilin (CEMA) und 0,1 Gewichtsteilen Hydrochinonmonomethylether zu einer homogenen Paste. Mit der Masse wurden Prüfkörper hergestellt, die dann mit Hilfe eines handelsüblichen dentalen Bestrahlungsgeräts 2 Minuten lang mit sichtbarem Licht einer Wellenlänge von > 400 nm ausgehärtet wurden. Die nach 24 Stunden bzw. 7 Tagen Wasserlagerung der Probekörper bestimmte Biegefestigkeit lag bei 66 bzw. 60 MPa, während der E-Modul 3100 bzw. 3380 MPa betrug. Dabei wurde ein Polymerisationsentwurf von 5,9 Vol.-% beobachtet und eine Durchhärtungstiefe von 4,9 mm erreicht.
   Mit den erfindungsgemäßen Enaminen sind somit in besonders geeigneter Weise Füllungskompositmaterialien zugänglich.
2) Elastisches Matrixpolymer für temporäre Füllungsmaterialien:
   Das Monomer la wurde zunächst mit dem Photoinitiatorsystem CC (0,3 Gew.-%) und CEMA (0,5 Gew.-%) versetzt, und man stellte damit durch Photopolymerisation Normstäbe für den Zugversuch (DIN 53504) her. Die Bestimmung der Reißfestigkeit bzw. Reißdehnung ergab Werte von 1,9 MPa bzw. 720 %, was die sehr guten elastischen Eigenschaften des Materials zum Ausdruck bringt.

Eine Variation der elastischen Eigenschaften war durch Copolymerisation mit geeigneten Vernetzermonomeren, wie z.B. TGDMA, sowie durch Zugabe entsprechender partikulärer Füllstoffe, wie z.B. hochdisperse Fällungskieselsäure oder Calciumhydroxid, möglich, so daß mit den erfindungsgemäßen Enaminen temporäre Füllungsmaterialien sowie Unterfütterungs- oder Unterfüllungsmaterialien erhalten werden konnten.

### Beispiel 8

### Polymeranaloge Umsetzungen der erfindungsgemäßen Verbindungen:

Durch radikalische Polymerisation von Methacryloxyethyl-3-N-butylamonocrotonat (Monomer **1a**; 1,0 mol/l) in Tetrahydrofuran (THF) in Gegenwart von Azobisisobutyronitril (AIBN) (0,05 mol/l) wurde nach 2 Stunden das entsprechende Poly(**1a**) mit einer zahlenmittleren Molmasse von 62800 g/mol (GPC, kalibriert mit PMMA-Standards) erhalten.

Die spektroskopische Charakterisierung des isolierten Polymeren zeigte, daß durch die Polymerisation des monomeren Enamins la nur die Signale der Methacrylat-Doppelbindung verschwunden waren, während die Enamin-Struktur erhalten blieb. So fehlten z.B. im ¹H-NMR-Spektrum von Poly(**1a**) die Signale der Vinylprotonen, und die Kernresonanzen der anderen Protonen, wie z.B. der NH-(8,60 ppm; 1H; t) (-CH=)- (4,50; 1H, s), (-CH₂-CH₂)- (4, 18; 4H; m) oder der CH₃-Gruppen (1,95; 6H; s/0,93; 3H, s) waren sichtbar. Darüber hinaus war auch im IR-Spektrum von Poly(**1a**) die Absorptionsbande der Valenzschwingung der Enaminbande bei 1606 cm⁻¹ vorhanden.

Von dem so erhaltenen Poly(**1a**) wurde eine 5 %ige Lösung in THF hergestellt, zu der man eine äquimolare Menge Acrylnitril und 0,05 Gew.-% Diazabicyclo[4.3.0]non-5-en (Katalysator) unter Rühren bei Raumtemperatur zugab. Nach weiteren 4 Stunden Rühren wurde das modifizierte Polymer in Methanol ausgefällt, mit Methanol gewaschen und bis zur Gewichtskonstanz im Feinvakuum getrocknet. Die Elementaranalyse des durch polymeranaloge Michael-Addition mit Acrylnitril modifiziertem Poly(**1a**) ergab einen Stickstoffgehalt von 7,72 %, woraus sich ein Umsetzungsgrad der Enamingruppen von 68,8 % berechnen läßt.

### Beispiel 9

### Herstellung einer festen Kunststoffmatrix über eine 2-Phasen-Härtung

Zu 10 g einer Mischung von acetoacetyliertem Bis-GMA (9,75 mmol) und TGDMA (17,46 mmol) fügte man als Photoinitiatorsystem CC (0,3 Gew.-%) und CEMA (0,5 Gew.-%) hinzu und tropfte unter schnellem Rühren eine äquimolare Menge Ethylendiamin (9,75 mmol) bei Raumtemperatur zu. Innerhalb von 10 Minuten stieg die bei 30°C bestimmte Viskosität der Ausgangsmischung von 190 mPas auf den über 63-fachen Wert von 12100 mPas an. Anschließend wurde die hochviskose Monomermasse 3 Minuten lang mit sichtbarem Licht einer Wellenlänge von > 400 nm bestrahlt, wobei ein fester Kunststoff mit einer Shore-D-Härte von 55 erhalten wurde.

## Patentansprüche

1. Polymerisierbare Verbindung der allgemeinen Formel (I), 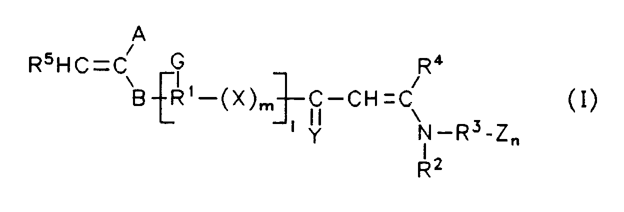 wobei
l eine ganze Zahl ≥ 1 ist,
m und n jeweils 0 oder 1 sind,
und
A Wasserstoff oder Alkyl,
B COO, CONH, C₆H₄ oder Arylen,
R¹ Alkylen, Oxyalkylen, C₆H₄ oder Arylen,
R² Wasserstoff, Alkyl oder Aryl,
R³ Wasserstoff, Alkyl oder Aryl bedeutet, wenn n = 0 ist,
R³ Alkylen oder Arylen bedeutet, wenn n = 1 ist,
wobei
R² und R³ nicht gleichzeitig Wasserstoff bedeuten,
R⁴ Alkyl,
R⁵ Wasserstoff, C₆H₅, CO₂H, CO₂Alkyl oder CN,
G Wasserstoff bedeutet,
X Sauerstoff, Schwefel oder Imino,
Y Sauerstoff oder Schwefel
bedeuten und
Z für eine oder mehrere funktionelle Gruppen steht, ausgenommen Verbindungen, bei denen die Gruppe -NR²R³Zₙ für -N(CH₃)₂, -N[CH(CH₃)₂]₂ oder -NH(CH₂CH₂OH) steht.

2. Verbindung nach Anspruch 1, dadurch gekennzeichnet, daß Alkyl und Alkylen für gesättigte und/oder ungesättigte Kohlenwasserstoff-Reste mit 1 bis 12 Kohlenstoffatomen stehen, wobei die Alkyl- und Alkylen-Gruppen untereinander jeweils gleich oder verschieden sein können.

3. Verbindung nach Anspruch 1, dadurch gekennzeichnet, daß Alkyl und Alkylen für gesättigte und/oder ungesättigte Kohlenwasserstoff-Reste mit mehr als 12 Kohlenstoffatomen stehen, wobei die Alkyl- und Alkylen-Gruppen untereinander jeweils gleich oder verschieden sein können.

4. Verbindung nach Anspruch 1 oder 2, dadurch gekennzeichnet, daß l = m = 1 und n = 0 ist und A Methyl, B COO, R¹ C₂H₄, R² Wasserstoff, R⁴ Methyl, R⁵ Wasserstoff, X und Y Sauerstoff bedeuten und R³ für einen Substituenten aus der Gruppe bestehend aus Butyl, Hexyl, Octyl und Dodecyl steht.

5. Verbindung nach Anspruch 1 oder 2, dadurch gekennzeichnet, daß l = m = 1 ist und A Methyl, B COO, R¹ C₂H₄, R² Wasserstoff, R⁴ Methyl, R⁵ Wasserstoff, X und Y Sauerstoff bedeuten und R³Z entweder 3-Hydroxypropyl oder 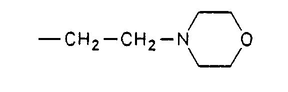 ist.

6. Verbindung nach Anspruch 1 oder 2, dadurch gekennzeichnet, daß l = 5, m = 1 und n = 0 ist und A Methyl, B COO, R¹ C₂H₄, R² Butyl, R³ Wasserstoff, R⁴ Methyl, R⁵ Wasserstoff und X und Y Sauerstoff bedeuten.

7. Verbindung nach Anspruch 1 oder 2, dadurch gekennzeichnet, daß l = m = n = 1 ist und A Methyl, B COO, R¹ C₂H₄, R² Wasserstoff, R⁴ Methyl, R⁵ Wasserstoff, X und Y Sauerstoff bedeuten und R³Z' der Gruppe -(CH₂)₄-CHZ-COOH entspricht, wobei Z' für die funktionelle Gruppe NH₂ oder für Wasserstoff steht.

8. Photopolymerisierbare Verbindung, bei der die Verbindung mindestens zwei Struktureinheiten der allgemeinen Formel (I) enthält, in welcher l, m, n, A, B, R¹, R³, R⁴, R⁵, X, Y und Z die oben angegebene Bedeutung haben, aber G und/oder R² die Struktureinheiten verknüpfende Brückensubstituenten sind.

9. Verbindung nach Anspruch 8, bei der die Verbindung zwei Struktureinheiten der allgemeinen Formel (I) umfaßt, in denen l = m = 1 und n = 0 ist, wobei A Methyl, B COO, R¹ C₂H₄, R³ Wasserstoff, R⁴ Methyl, R⁵ Wasserstoff und X und Y Sauerstoff bedeuten und R² für den beide Struktureinheiten verknüpfenden Brückensubstituenten -CH₂-(CH₂)ᵣ-CH₂- steht, wobei r entweder 0, 1, 4 oder 8 ist.

10. Verbindung nach Anspruch 8, bei der die Verbindung zwei Struktureinheiten der allgemeinen Formel (I) umfaßt, wobei l = m = 1 und n = 0 ist, A Methyl, B COO, R¹ C₂H₄, R³ Wasserstoff, R⁴ Methyl, R⁵ Wasserstoff und X und Y Sauerstoff bedeuten und R² für den beide Struktureinheiten verknüpfenden Brückensubstituenten 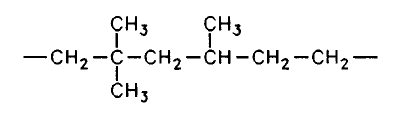 steht.

11. Verbindung nach Anspruch 8, bei der die Verbindung zwei Struktureinheiten der allgemeinen Formel (I) umfaßt, wobei l = m = 1 und n = 0 ist, A Methyl, B COO, R¹ C₂H₄, R³ Wasserstoff, R⁴ Methyl, R⁵ Wasserstoff und X und Y Sauerstoff bedeuten und R² für den Brückensubstituenten 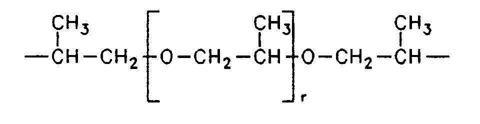 steht, wobei r ≥ 1 ist.

12. Verbindung nach Anspruch 11, wobei r = 5 ist.

13. Verbindung nach einem der Ansprüche 1 bis 12, wobei die polymerisierbare Verbindung ein Monomer ist.

14. Verbindung nach Anspruch 8, wobei die polymerisierbare Verbindung ein Präpolymer ist.

15. Verfahren zur Herstellung der Verbindung nach einem der Ansprüche 1, 2, 3 oder 8, bei dem man die Verbindung der allgemeinen Formel (II) 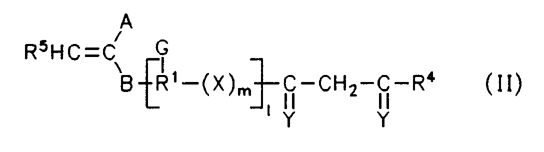 mit einem primären Amin der allgemeinen Formel NH₂R², mit einem sekundären Amin der allgemeinen Formel NHR²R³, mit einem funktionalisierten Amin der allgemeinen Formel NHR²(R³Zₙ) oder mit einem Diamin der allgemeinen Formel H(R³)N-R²-N(R³)H oder H(R³Zₙ)N-R²-N(R³Zₙ)H umsetzt, wobei l, m, n, A, B, R¹, R², R³, R⁴, R⁵, X, Y, Z und G die oben angegebene Bedeutung haben, ausgenommen NH(CH₃)₂, NH[CH(CH₃)₂]₂ oder NH₂(CH₂CH₂OH).

16. Verfahren nach Anspruch 15, wobei die Verbindung der allgemeinen Formel (II) 2-Acetoacetoxyethylmethacrylat ist.

17. Verfahren nach Anspruch 15, wobei die Verbindung der allgemeinen Formel (II) PEG-200-(2-Acetoacetoxymethacrylat) ist.

18. Verfahren nach einem der Ansprüche 15 bis 17, wobei als primäres Amin Butylamin, Hexylamin, Octylamin oder Dodecylamin verwendet wird.

19. Verfahren nach einem der Ansprüche 15 bis 17, wobei als funktionalisiertes Amin 3-Amino-propan-1-ol verwendet wird.

20. Verfahren nach einem der Ansprüche 15 bis 17, wobei als funktionalisiertes Amin 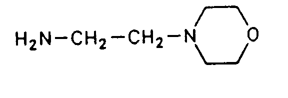 verwendet wird.

21. Verfahren nach einem der Ansprüche 15 bis 17, wobei als funktionalisiertes Amin eine Verbindung der allgemeinen Formel (III) 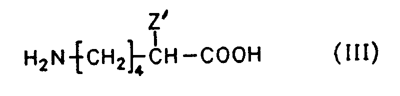 verwendet wird, wobei Z' Wasserstoff oder NH₂ bedeutet.

22. Verfahren nach Anspruch 15, wobei die Verbindung der allgemeinen Formel (II) aus zwei 2-Acetoacetoxyethylmethacrylat-Gruppen besteht, wobei der Brückensubstituent G 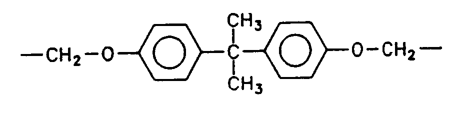 ist.

23. Verfahren nach Anspruch 15, dadurch gekennzeichnet, daß als Diamin eine Verbindung der allgemeinen Formel (IV)
H₂N-R²-NH₂ (IV)
verwendet wird, wobei
der Brückensubstituent R² aus der Gruppe bestehend aus -CH₂-(CH₂)ᵣ-CH₂-,-CH₂-C(CH₃)₂CH₂CH(CH₃)CH₂-CH₂- oder -CH(CH₃)-CH₂(OCH₂CH(CH₃))_{q}OCH₂-CH(CH₃)- ausgewählt wird, wobei r 0, 1, 4 oder 8 und q eine ganze Zahl größer 1 ist.

24. Verwendung einer polymerisierbaren Verbindung mit mindestens einer Struktureinheit der allgemeinen Formel (I), 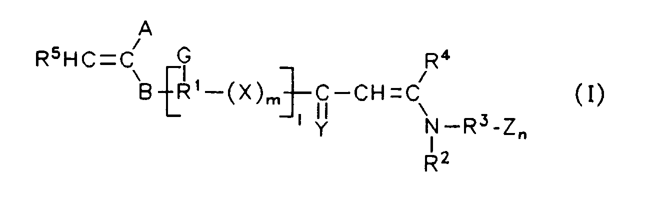 wobei
l eine ganze Zahl ≥ 1 ist,
m und n jeweils 0 oder 1 sind,
und
A Wasserstoff oder Alkyl,
B COO, CONH, C₆H₄ oder Arylen,
R¹ Alkylen, Oxyalkylen, C₆H₄ oder Arylen,
R² Wasserstoff, Alkyl oder Aryl,
R³ Wasserstoff, Alkyl oder Aryl bedeutet, wenn n = 0 ist,
R³ Alkylen oder Arylen bedeutet, wenn n = 1 ist,
wobei
R² und R³ nicht gleichzeitig Wasserstoff bedeuten,
R⁴ Alkyl,
R⁵ Wasserstoff, C₆H₅, CO₂H, CO₂Alkyl oder CN,
G Wasserstoff bedeutet,
X Sauerstoff, Schwefel oder Imino,
Y Sauerstoff oder Schwefel
bedeuten und
Z für eine oder mehrere funktionelle Gruppen steht,
zur Herstellung von Dentalmaterialien.

25. Verwendung nach Anspruch 24, dadurch gekennzeichnet, daß Alkyl und Alkylen für gesättigte und/oder ungesättigte Kohlenwasserstoff-Reste mit 1 bis 12 Kohlenstoffatomen stehen, wobei die Alkyl- und Alkylen-Gruppen untereinander jeweils gleich oder verschieden sein können.

26. Verwendung nach Anspruch 24, dadurch gekennzeichnet, daß Alkyl und Alkylen für gesättigte und/oder ungesättigte Kohlenwasserstoff-Reste mit mehr als 12 Kohlenstoffatomen stehen, wobei die Alkyl- und Alkylen-Gruppen untereinander jeweils gleich oder verschieden sein können.

27. Verwendung nach Anspruch 24 oder 25, dadurch gekennzeichnet, daß l = m = 1 und n = 0 ist und A Methyl, B COO, R¹ C₂H₄, R² Wasserstoff, R⁴ Methyl, R⁵ Wasserstoff, X und Y Sauerstoff bedeuten und R³ für einen Substituenten aus der Gruppe bestehend aus Butyl, Hexyl, Octyl und Dodecyl steht.

28. Verwendung nach Anspruch 24 oder 25, dadurch gekennzeichnet, daß l = m = 1 ist und A Methyl, B COO, R¹ C₂H₄, R² Wasserstoff, R⁴ Methyl, R⁵ Wasserstoff, X und Y Sauerstoff bedeuten und R³Z entweder 3-Hydroxypropyl oder 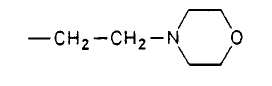 ist.

29. Verwendung nach Anspruch 24 oder 25, dadurch gekennzeichnet, daß l = 5, m = 1 und n = 0 ist und A Methyl, B COO, R¹ C₂H₄, R² Butyl, R³ Wasserstoff, R⁴ Methyl, R⁵ Wasserstoff und X und Y Sauerstoff bedeuten.

30. Verwendung nach Anspruch 24 oder 25, dadurch gekennzeichnet, daß l = m = n = 1 ist und A Methyl, B COO, R¹ C₂H₄, R² Wasserstoff, R⁴ Methyl, R⁵ Wasserstoff, X und Y Sauerstoff bedeuten und R³Z' der Gruppe -(CH₂)₄-CHZ'-COOH entspricht, wobei Z für die funktionelle Gruppe NH₂ oder für Wasserstoff steht.

31. Verwendung nach Anspruch 24, bei der die Verbindung mindestens zwei Struktureinheiten der allgemeinen Formel (I) enthält, in welcher l, m, n, A, B, R¹, R³, R⁴, R⁵, X, Y und Z die oben angegebene Bedeutung haben, aber G und/oder R² die Struktureinheiten verknüpfende Brückensubstituenten sind.

32. Verwendung nach Anspruch 31, dadurch gekennzeichnet, daß der Brückensubstituent G 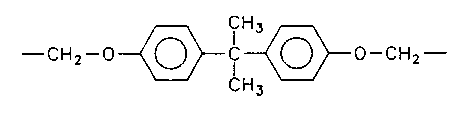 ist.

33. Verwendung nach Anspruch 31, dadurch gekennzeichnet, daß der Brückensubstituent R² aus der Gruppe bestehend aus -CH₂-(CH₂)ᵣ-CH₂-, -CH₂-C(CH₃)₂CH₂CH(CH₃)CH₂-CH₂- oder -CH(CH₃)-CH₂(OCH₂CH(CH₃))_{q}OCH₂-CH(CH₃)-ausgewählt wird, wobei r 0, 1, 4 oder 8 und q eine ganze Zahl größer 1 ist.

34. Verwendung nach Anspruch 31, bei der die Verbindung zwei Struktureinheiten der allgemeinen Formel (I) umfaßt, in denen l = m = 1 und n = 0 ist, wobei A Methyl, B COO, R¹ C₂H₄, R³ Wasserstoff, R⁴ Methyl, R⁵ Wasserstoff und X und Y Sauerstoff bedeuten und R² für den beide Struktureinheiten verknüpfenden Brückensubstituenten -CH₂-(CH₂)ᵣ-CH₂- steht, wobei r entweder 0, 1, 4 oder 8 ist.

35. Verwendung nach Anspruch 31, bei der die Verbindung zwei Struktureinheiten der allgemeinen Formel (I) umfaßt, wobei l = m = 1 und n = 0 ist, A Methyl, B COO, R¹ C₂H₄, R³ Wasserstoff, R⁴ Methyl, R⁵ Wasserstoff und X und Y Sauerstoff bedeuten und R² für den beide Struktureinheiten verknüpfenden Brückensubstituenten 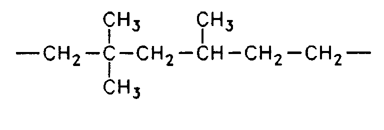 steht.

36. Verwendung nach Anspruch 31, bei der die Verbindung zwei Struktureinheiten der allgemeinen Formel (I) umfaßt, wobei l = m = 1 und n = 0 ist, A Methyl, B COO, R¹ C₂H₄, R³ Wasserstoff, R⁴ Methyl, R⁵ Wasserstoff und X und Y Sauerstoff bedeuten und R² für den Brückensubstituenten 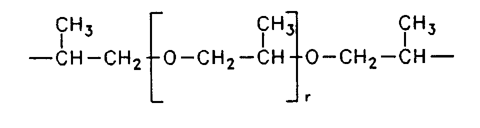 steht, wobei r ≥ 1 ist.

37. Verwendung nach Anspruch 36, wobei r = 5 ist.

38. Verwendung nach einem der Ansprüche 24 bis 37, wobei die polymerisierbare Verbindung ein Monomer ist.

39. Verwendung nach Anspruch 31, wobei die polymerisierbare Verbindung ein Präpolymer ist.

40. Verwendung nach einem der Ansprüche 24 bis 39, wobei das Dentalmaterial ein Homo- oder Copolymer ist.

41. Verwendung nach einem der Ansprüche 24 bis 39, wobei das Dentalmaterial ein Komposit-Material ist.

42. Verwendung nach einem der Ansprüche 24 bis 39, wobei das Dentalmaterial ein Abformmaterial, ein Füllungsmaterial, ein temporäres Füllungsmaterial, ein Unterfüllungsmaterial oder ein Unterfütterungsmaterial ist.

43. Verwendung nach einem der Ansprüche 24 bis 42, wobei die Verbindung durch Strahlung polymerisiert wird.

44. Verwendung der Verbindung nach einem der Ansprüche 8 bis 12 und 14 als Vernetzer, wobei G Wasserstoff ist und die Struktureinheiten der allgemeinen Formel (I) über Brückensubstituenten R² miteinander verknüpft sind.

## Claims

1. Polymerizable compound of the general formula (I) 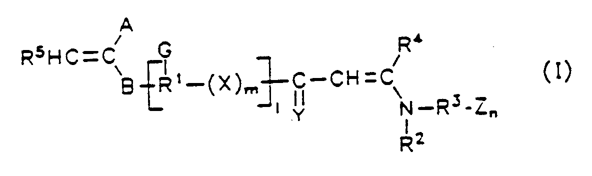 where
l is a whole number ≥ 1,
m and n are each 0 or 1,
and
A is hydrogen or alkyl,
B is COO, CONH, C₆H₄ or arylene,
R¹ is alkylene, oxyalkylene, C₆H₄ or arlylene,
R² is hydrogen, alkyl or aryl,
R³ is hydrogen, alkyl or aryl when n = 0,
R³ is alkylene or arylene when n = 1,
where
R² and R³ are not hydrogen at the same time,
R⁴ is alkyl,
R⁵ is hydrogen, C₆H₅, CO₂H, CO₂alkyl or CN,
G is hydrogen,
X is oxygen, sulphur or imino,
Y is oxygen or sulphur
and
Z is one or more functional groups,
with the exception of compounds in which the group -NR²R³Zₙ is -N(CH₃)₂, -N[CH(CH₃)₂]₂ or -NH(CH₂CH₂OH).

2. Compound according to Claim 1, characterized in that alkyl and alkylene are saturated and/or unsaturated hydrocarbon radicals having 1 to 12 carbon atoms, where the alkyl and alkylene groups can be the same as or different from one another in each case.

3. Compound according to Claim 1, characterized in that alkyl and alkylene are saturated and/or unsaturated hydrocarbon radicals having more than 12 carbon atoms, where the alkyl and alkylene groups can be the same as or different from one another in each case.

4. Compound according to Claim 1 or 2, characterized in that l = m = 1 and n = 0 and A is methyl, B is COO, R¹ is C₂H₄, R² is hydrogen, R⁴ is methyl, R⁵ is hydorgen, X and Y are oxygen and R³ is a substituent from the group consisting of butyl, hexyl, octyl and dodecyl.

5. Compound according to Claim 1 or 2, characterized in that l = m = 1 and A is methyl, B is COO, R¹ is C₂H₄, R² is hydrogen, R⁴ is methyl, R⁵ is hydrogen, X and Y are oxygen and R³Z is either 3-hydroxypropyl or

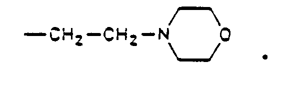

6. Compound according to Claim 1 or 2, characterized in that l = 5, m = 1 and n = 0 and A is methyl, B is COO, R¹ is C₂H₄, R² is butyl, R³ is hydrogen, R⁴ is methyl, R⁵ is hydrogen and X and Y are oxygen.

7. Compound according to Claim 1 or 2, characterized in that l = m = n = 1 and A is methyl, B is COO, R¹ is C₂H₄, R² is hydrogen, R⁴ is methyl, R⁵ is hydrogen, X and Y are oxygen and R³Z' corresponds to the group -(CH₂)₄-CHZ-COOH, where Z' is the functional group NH₂ or is hydrogen.

8. Photopolymerizable compound, where the compound contains at least two structural units of the general formula (I) in which l, m, n, A, B, R¹, R³, R⁴, R⁵, X, Y and Z have the meaning given above but G and/or R² are bridging substituents linking the structural units.

9. Compound according to Claim 8, where the compound includes two structural units of the general formula (I) in which l = m = 1 and n = 0, where A is methyl, B is COO, R¹ is C₂H₄, R³ is hydrogen, R⁴ is methyl, R⁵ is hydrogen and X and Y are oxygen and R² represents the bridging substituent -CH₂-(CH₂)ᵣ-CH₂- linking both structural units, where r is either 0, 1, 4 or 8.

10. Compound according to Claim 8, where the compound includes two structural units of the general formula (I) in which l = m = 1 and n = 0, A is methyl, B is COO, R¹ is C₂H₄, R³ is hydrogen, R⁴ is methyl, R⁵ is hydrogen and X and Y are oxygen and R² is the bridging constituent 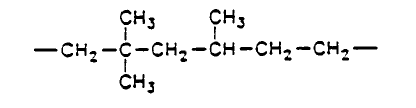 linking both structural units.

11. Compound according to Claim 8, where the compound includes two structural units of the general formula (I) in which l = m = 1 and n = 0, A is methyl, B is COO, R¹ is C₂H₄, R³ is hydrogen, R⁴ is methyl, R⁵ is hydrogen and X and Y are oxygen and R² is the bridging constituent 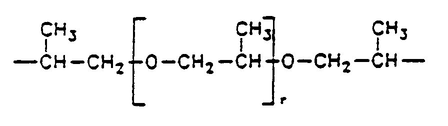 where r is ≥ 1.

12. Compound according to Claim 11, where r = 5.

13. Compound according to one of Claims 1 to 12, where the polymerizable compound is a monomer.

14. Compound according to Claim 8, where the polymerizable compound is a prepolymer.

15. Process for the preparation of the compound according to one of Claims 1, 2, 3 or 8, in which the compound of the general formula (II) 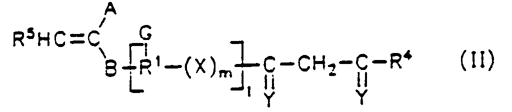 is reacted with a primary amine of the general formula NH₂R², with a secondary amine of the general formula NHR²R³, with a functionalized amine of the general formula NHR²(R³Zₙ) or with a diamine of the general formula H(R³)N-R²-N(R³)H or H(R³Zₙ)N-R²-N(R³Zₙ)H, where l, m, n, A, B, R¹, R², R³, R⁴, R⁵, X, Y, Z and G have the meaning given above, with the exception of NH(CH₃)₂, NH[CH(CH₃)₂]₂ or NH₂(CH₂CH₂OH).

16. Process according to Claim 15, where the compound of the general formula (II) is 2-acetoacetoxyethyl methacrylate.

17. Process according to Claim 15, where the compound of the general formula (II) is PEG 200-(2-acetoacetoxy methacrylate).

18. Process according to one of Claims 15 to 17, where butylamine, hexylamine, octylamine or dodecylamine is used as primary amine.

19. Process according to one of Claims 15 to 17, where 3-aminopropan-1-ol is used as functionalized amine.

20. Process according to one of Claims 15 to 17, where 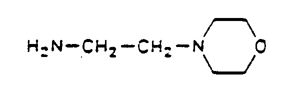 is used as functionalized amine.

21. Process according to one of Claims 15 to 17, where a compound of the general formula (III) 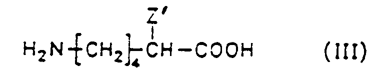 where Z' is hydrogen or NH₂ is used as functionalized amine.

22. Process according to Claim 15, where the compound of the general formula (II) consists of two 2-acetoacetoxyethyl methacrylate groups, where the bridging substituent G is

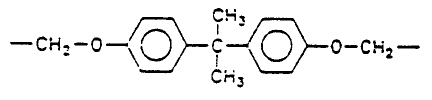

23. Process according to Claim 15, characterized in that as diamine a compound of the general formula (IV)
H₂N-R²-NH₂ (IV)
is used, where
the bridging substituent R² is selected from the group consisting of -CH₂-(CH₂)ᵣ-CH₂-, -CH₂-C(CH₃)₂CH₂CH(CH₃)CH₂-CH₂- or -CH(CH₃)-CH₂(OCH₂CH(CH₃))_{q}OCH₂-CH(CH₃)-, where r is 0, 1, 4 or 8 and q is a whole number greater than 1.

24. Use of a polymerizable compound having at least one structural unit of the general formula (I) 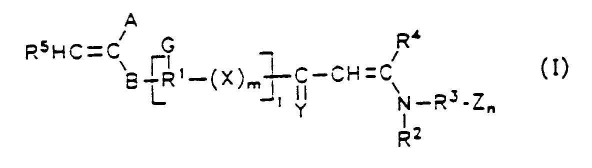 where
l is a whole number ≥ 1,
m and n are each 0 or 1,
and
A is hydrogen or alkyl,
B is COO, CONH, C₆H₄ or arylene,
R¹ is alkylene, oxyalkylene, C₆H₄ or arlylene,
R² is hydrogen, alkyl or aryl,
R³ is hydrogen, alkyl or aryl when n = 0,
R³ is alkylene or arylene when n = 1,
where
R² and R³ are not hydrogen at the same time,
R⁴ is alkyl,
R⁵ is hydrogen, C₆H₅, CO₂H, CO₂alkyl or CN,
G is hydrogen,
X is oxygen, sulphur or imino,
Y is oxygen or sulphur
and
Z is one or more functional groups
for the production of dental materials.

25. Use according to Claim 24, characterized in that alkyl and alkylene are saturated and/or unsaturated hydrocarbon radicals having 1 to 12 carbon atoms, where the alkyl and alkylene groups can be the same as or different from one another in each case.

26. Use according to Claim 24, characterized in that alkyl and alkylene are saturated and/or unsaturated hydrocarbon radicals having more than 12 carbon atoms, where the alkyl and alkylene groups can be the same as or different from one another in each case.

27. Use according to Claim 24 or 25, characterized in that l = m = 1 and n = 0 and A is methyl, B is COO, R¹ is C₂H₄, R² is hydrogen, R⁴ is methyl, R⁵ is hydorgen, X and Y are oxygen and R³ is a substituent from the group consisting of butyl, hexyl, octyl and dodecyl.

28. Use according to Claim 24 or 25, characterized in that l = m = 1 and A is methyl, B is COO, R¹ is C₂H₄, R² is hydrogen, R⁴ is methyl, R⁵ is hydrogen, X and Y are oxygen and R³Z is either 3-hydroxypropyl or

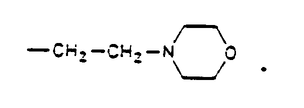

29. Use according to Claim 24 or 25, characterized in that l = 5, m = 1 and n = 0 and A is methyl, B is COO, R¹ is C₂H₄, R² is butyl, R³ is hydrogen, R⁴ is methyl, R⁵ is hydrogen and X and Y are oxygen.

30. Use according to Claim 24 or 25, characterized in that l = m = n = 1 and A is methyl, B is COO, R¹ is C₂H⁴, R² is hydrogen, R⁴ is methyl, R⁵ is hydrogen, X and Y are oxygen and R³Z' corresponds to the group -(CH₂)₄-CH₄-COOH, where Z' is the functional group NH₂ or is hydrogen.

31. Use according to Claim 24, where the compound contains at least two structural units of the general formula (I) in which l, m, n, A, B, R¹, R³, R⁴, R⁵, X, Y and Z have the meaning given above but G and/or R² are bridging substituents linking the structural units.

32. Use according to Claim 31, characterized in that the bridging substituent G is

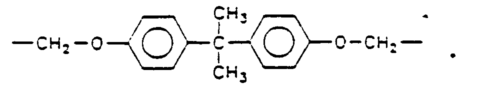

33. Use according to Claim 31, characterized in that the bridging substituent R² is selected from the group consisting of -CH₂-(CH₂)ᵣ-CH₂-, -CH₂-C(CH₃)₂CH₂CH(CH₃)CH₂-CH₂- or -CH(CH₃)-CH₂(OCH₂CH(CH₃))_{q}OCH₂-CH(CH₃)-, where r is 0, 1, 4 or 8 and q is a whole number greater than 1.

34. Use according to Claim 31, where the compound includes two structural units of the general formula (I) in which l = m = 1 and n = 0, where A is methyl, B is COO, R¹ is C₂H₄, R³ is hydrogen, R⁴ is methyl, R⁵ is hydrogen and X and Y are oxygen and R² represents the bridging substituent -CH₂-(CH₂)ᵣ-CH₂- linking both structural units, where r is either 0, 1, 4 or 8.

35. Use according to Claim 31, where the compound includes two structural units of the general formula (I) in which l = m = 1 and n = 0, A is methyl, B is COO, R¹ is C₂H₄, R³ is hydrogen, R⁴ is methyl, R⁵ is hydrogen and X and Y are oxygen and R² is the bridging constituent 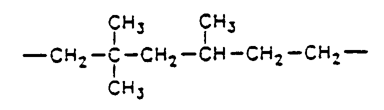 linking both structural units.

36. Use according to Claim 31, where the compound includes two structural units of the general formula (I) in which l = m = 1 and n = 0, A is methyl, B is COO, R¹ is C₂H₄, R³ is hydrogen, R⁴ is methyl, R⁵ is hydrogen and X and Y are oxygen and R² is the bridging constituent 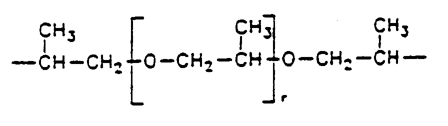 where r is ≥ 1.

37. Use according to Claim 36, where r = 5.

38. Use according to one of Claims 24 to 37, where the polymerizable compound is a monomer.

39. Use according to Claim 31, where the polymerizable compound is a prepolymer.

40. Use according to one of Claims 24 to 39, where the dental material is a homopolymer or copolymer.

41. Use according to one of Claims 24 to 39, where the dental material is a composite material.

42. Use according to one of Claims 24 to 39, where the dental material is an impression material, a filling material, a temporary filling material, a relining material or an interfacing material.

43. Use according to one of Claims 24 to 42, where the compound is polymerized by radiation.

44. Use of the compound according to one of Claims 8 to 12 and 14 as crosslinking agent, where G is hydrogen and the structural units of the general formula (I) are linked to one another by way of bridging substituents R².

## Revendications

1. Composés polymérisables de formule générale (I) 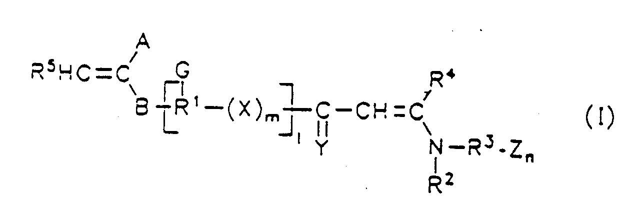 dans laquelle,
l est un nombre entier ≥ 1,
m et n sont respectivement 0 ou 1,
et
A est un atome d'hydrogène ou un groupe alkyle,
B est un groupe COO, CONH, C₆H₄ ou arylène,
R¹ est un groupe alkylène, oxyalkylène, C₆H₄ ou arylène,
R² est un atome d'hydrogène, un groupe alkyle ou aryle,
R³ représente un atome d'hydrogène, un groupe alkyle ou aryle, quand n = 0,
R³ représente un groupe alkylène ou arylène, quand n = 1,
dans laquelle,
R² et R³ ne représentent pas en même temps l'atome d'hydrogène,
R⁴ représente un groupe alkyle,
R⁵ représente un atome d'hydrogène, un groupe C₆H₅, CO₂H, CO₂alkyle ou CN,
G représente un atome d'hydrogène,
X un atome d'oxygène, de soufre ou un groupe imino,
Y un atome d'oxygène ou de soufre
et
Z représente un ou plusieurs groupes fonctionnels, excepté les composés dans lesquels le groupe -NR²R³Zₙ représente -N(CH₃)₂, -N[CH(CH₃)₂]₂ ou -NH(CH₂CH₂OH).

2. Composé selon la revendication 1, caractérisé en ce que les groupes alkyle ou alkylène représentent des radicaux hydrocarbonés saturés ou insaturés avec entre 1 et 12 atomes de carbone, les groupes alkyle et alkylène pouvant être respectivement égaux ou différents entre eux.

3. Composé selon la revendication 1, caractérisé en ce que les groupes alkyle ou alkylène représentent des radicaux hydrocarbonés saturés ou insaturés avec plus de 12 atomes de carbone, les groupes alkyle et alkylène pouvant être respectivement égaux ou différents entre eux.

4. Composé selon la revendication 1 ou 2, caractérisé en ce que l = m = 1 et n = 0 et A représente un groupe méthyle, B COO, R¹ C₂H₄, R² un atome d'hydrogène, R⁴ un groupe méthyle, R⁵ un atome d'hydrogène, X et Y un atome d'oxygène et R³ représente un substituant choisi dans le groupe constitué du groupe butyle, hexyle, octyle, et dodécyle.

5. Composé selon la revendication 1 ou 2, caractérisé en ce que l = m = 1 et n = 0 et A représente un groupe méthyle, B COO, R¹ C₂H₄, R² un atome d'hydrogène, R⁴ un groupe méthyle, R⁵ un atome d'hydrogène, X et Y un atome d'oxygène et R³Z est soit le 3-hydroxypropyle ou

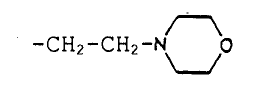

6. Composé selon la revendication 1 ou 2, caractérisé en ce que l = 5, m = 1 et n = 0 et A représente un groupe méthyle, B COO, R¹ C₂H₄, R² un groupe butyle, R³ un atome d'hydrogène, R⁴ un groupe méthyle, R⁵ un atome d'hydrogène, X et Y un atome d'oxygène.

7. Composé selon la revendication 1 ou 2, caractérisé en ce que l = m = n = 1 et A représente un groupe méthyle, B COO, R¹ C₂H₄, R² un atome d'hydrogène, R⁴ un groupe méthyle, R⁵ un atome d'hydrogène, X et Y un atome d'oxygène et R³Z' correspond au groupe -(CH₂)₄-CHZ'-COOH, Z' représentant un groupe fonctionnel NH₂ ou un atome d'hydrogène.

8. Composé photopolymérisable, dans lequel le composé contient au moins deux motifs de structure de formule générale (I), dans laquelle l, m, n, A, B, R¹, R³, R⁴, R⁵, X, Y, et Z ont la signification donnée ci-dessus, mais G et/ou R² sont les substituants pontants reliant les motifs de structure.

9. Composé selon la revendication 8, dans lequel le composé contient au moins deux motifs de structure de formule générale (I), dans lesquels l = m = 1 et n = 0, A représentant un groupe méthyle, B COO, R¹ C₂H₄, R³ un atome d'hydrogène, R⁴ un groupe méthyle, R⁵ un atome d'hydrogène et X et Y un atome d'oxygène et R² représentant pour les substituants pontants reliant les deux motifs de structure -CH₂-(CH₂)ᵣ-CH₂-, r étant 0, 1, 4 ou 8.

10. Composé selon la revendication 8, dans lequel le composé contient au moins deux motifs de structure de formule générale (I), l étant = m = 1 et n = 0, A représentant un groupe méthyle, B COO, R¹ C₂H₄, R³ un atome d'hydrogène, R⁴ un groupe méthyle, R⁵ un atome d'hydrogène et X et Y un atome d'oxygène et R² représentant pour les substituants pontants reliant les deux motifs de structure

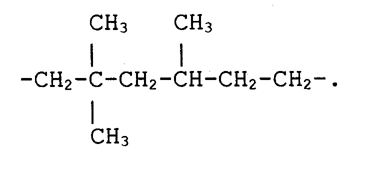

11. Composé selon la revendication 8, dans lequel le composé contient au moins deux motifs de structure de formule générale (I), l étant = m = 1 et n = 0, A représentant un groupe méthyle, B COO, R¹ C₂H₄, R³ un atome d'hydrogène, R⁴ un groupe méthyle, R⁵ un atome d'hydrogène et X et Y un atome d'oxygène et R² représentant le substituant pontant 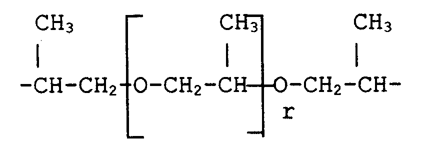 dans lequel r ≥ 1.

12. Composé selon la revendication 11, dans lequel r = 5.

13. Composé selon une des revendications 1 à 12, le composé polymérisable étant un monomère.

14. Composé selon la revendication 8, dans lequel le composé polymérisable est un prépolymère.

15. Procédé de préparation du composés selon une des revendications 1, 2, 3 ou 8, dans lequel on fait réagir le composé de formule générale (II) 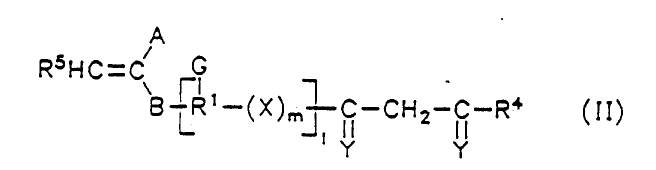 avec une amine primaire de formule générale NH₂R², avec une amine secondaire de formule générale NHR²R³, avec une amine fonctionnalisée de formule générale NHR²(R³Zₙ) ou avec une diamine de formule générale H(R³)N-R²-N(R³)H ou H(R³Zₙ)N-R²-N(R³Zₙ)H, l, m, n, A, B, R¹, R², R³, R⁴, R⁵, X, Y, Z et G ayant la signification donnée ci-dessus, à l'exception de NH(CH₃)₂, NH[CH(CH₃)₂]₂ ou NH₂(CH₂CH₂OH).

16. Composé selon la revendication 15, dans lequel le composé de formule générale (II) est le méthacrylate de 2-acétoacétoxyéthyle.

17. Composé selon la revendication 15, dans lequel le composé de formule générale (II) est le (méthacrylate de 2-acétoacétoxy)-PEG-200.

18. Procédé selon une des revendications 15 à 17, dans lequel on utilise comme amine primaire la butylamine, l'hexylamine, l'octylamine ou la dodécylamine.

19. Procédé selon une des revendications 15 à 17, dans lequel on utilise comme amine fonctionnalisée le 3-amino-propan-1-ol.

20. Procédé selon une des revendications 15 à 17, dans lequel on utilise comme amine fonctionnalisée

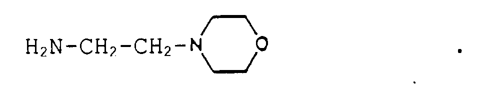

21. Procédé selon une des revendications 15 à 17, dans lequel on utilise comme amine fonctionnalisée un composé de formule générale (III) 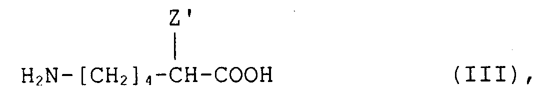 Z' représentant un atome d'hydrogène ou un groupe NH₂.

22. Procédé selon la revendication 15, dans lequel le composé de formule générale (II) est constitué de deux groupes méthacrylate de 2-acétoacétoxyéthyle, le substituant pontant G étant

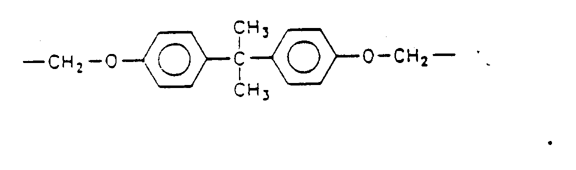

23. Procédé selon la revendication 15, caractérisé en ce qu'on utilise comme diamine un composé de formule générale (IV)
H₂N-R²-NH₂, (IV)
dans lequel
le substituant pontant R² est choisi parmi le groupe des radicaux constitué de -CH₂-(CH₂)ᵣ-CH₂-, -CH₂-C(CH₃)₂-CH₂-CH(CH₃)CH₂-CH₂- ou
-CH(CH₃)-CH₂(OCH₂CH(CH₃))_{q}OCH₂-CH(CH₃), r étant 0, 1, 4 ou 8 et q un nombre entier supérieur à 1.

24. Utilisation d'un composé polymérisable avec au moins un motif de structure de formule générale (I) 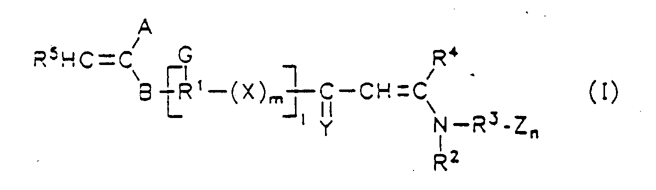 dans laquelle,
l est un nombre entier ≥ 1,
m et n sont respectivement 0 ou 1,
et
A est un atome d'hydrogène ou un groupe alkyle,
B est un groupe COO, CONH, C₆H₄ ou arylène,
R¹ est un groupe alkylène, oxyalkylène, C₆H₄ ou arylène,
R² est un atome d'hydrogène, un groupe alkyle ou aryle,
R³ représente un atome d'hydrogène, un groupe alkyle ou aryle, quand n = 0,
R³ représente un groupe alkylène ou arylène, quand n = 1,
dans laquelle,
R² et R³ ne représentent pas en même temps l'atome d'hydrogène,
R⁴ représente un groupe alkyle,
R⁵ représente un atome d'hydrogène, un groupe C₆H₅, CO₂H, CO₂alkyle ou CN,
G représente un atome d'hydrogène,
X un atome d'oxygène, de soufre ou un groupe imino,
Y un atome d'oxygène ou de soufre
et
Z représente un ou plusieurs groupes fonctionnels, pour la préparation de matériaux dentaires.

25. Utilisation d'un composé polymérisable selon la revendication 24, caractérisée en ce que les groupes alkyle et alkylène représentent des radicaux hydrocar-bonés saturés et/ou insaturés avec entre 1 et 12 atomes de carbone, les groupes alkyle ou alkylène pouvant être respectivement égaux ou différents entre eux.

26. Utilisation d'un composé polymérisable selon la revendication 24, caractérisée en ce que les groupes alkyle et alkylène représentent des radicaux hydrocar-bonés saturés et/ou insaturés avec plus de 12 atomes de carbone, les groupes alkyle ou alkylène pouvant être respectivement égaux ou différents entre eux.

27. Utilisation d'un composé polymérisable selon la revendication 24 ou 25, caractérisée en ce que l = m = 1 et n = 0 et A représente un groupe méthyle, B COO, R¹ C₂H₄, R² un atome d'hydrogène, R⁴ un groupe méthyle, R⁵ un atome d'hydrogène, X et Y un atome d'oxygène et R³ représente un substituant choisi dans le groupe constitué du groupe butyle, hexyle, octyle, et dodécyle.

28. Utilisation d'un composé polymérisable selon la revendication 24 ou 25, caractérisé en ce que l = m = 1 et A représente un groupe méthyle, B COO, R¹ C₂H₄, R² un atome d'hydrogène, R⁴ un groupe méthyle, R⁵ un atome d'hydrogène, X et Y un atome d'oxygène et R³Z est soit le 3-hydroxypropyle ou

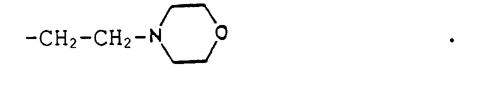

29. Utilisation d'un composé polymérisable selon la revendication 24 ou 25, caractérisée en ce que l = 5, m = 1 et n = 0 et A représente un groupe méthyle, B COO, R¹ C₂H₄, R² un groupe butyle, R³ un atome d'hydrogène, R⁴ un groupe méthyle, R⁵ un atome d'hydrogène, X et Y un atome d'oxygène.

30. Utilisation d'un composé polymérisable selon la revendication 24 ou 25, caractérisée en ce que l = m = n = 1 et A représente un groupe méthyle, B COO, R¹ C₂H₄, R² un atome d'hydrogène, R⁴ un groupe méthyle, R⁵ un atome d'hydrogène, X et Y un atome d'oxygène et R³Z' correspond au groupe -(CH₂)₄-CHZ'-COOH, Z' représentant le groupe fonctionnel NH₂ ou un atome d'hydrogène.

31. Utilisation d'un composé polymérisable selon la revendication 24, dans laquelle le composé contient au moins deux motifs de structure de formule générale (I), dans laquelle l, m, n, A, B, R¹, R³, R⁴, R⁵, X, Y, et Z ont la signification donnée ci-dessus, mais G et/ou R² sont des substituants pontants reliant les motifs de structure.

32. Utilisation d'un composé polymérisable selon la revendication 31, caractérisée en ce que le substituant pontant G est

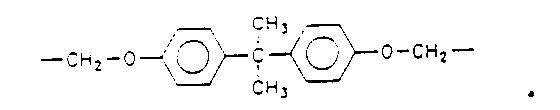

33. Utilisation d'un composé polymérisable selon la revendication 31, caractérisée en ce que le substituant pontant R² est choisi parmi le groupe des radicaux constitué de -CH₂-(CH₂)ᵣ-CH₂-, -CH₂-C(CH₃)₂CH₂CH(CH₃)CH₂-CH₂- ou
-CH(CH₃)-CH₂(OCH₂CH(CH₃))_{q}OCH₂-CH(CH₃), r étant 0, 1, 4 ou 8 et q un nombre entier supérieur à 1.

34. Utilisation d'un composé polymérisable selon la revendication 31, dans laquelle le composé contient deux motifs de structure de formule générale (I), dans lesquels l = m = 1 et n = 0, A représentant un groupe méthyle, B COO, R¹ C₂H₄, R² un atome d'hydrogène, R⁴ un groupe méthyle, R⁵ un atome d'hydrogène et X et Y un atome d'oxygène et R³ un substituant pontant
-CH₂(CH₂)ᵣ-CH₂- reliant les deux motifs, r étant 0, 1, 4 ou 8.

35. Utilisation d'un composé polymérisable selon la revendication 31, dans laquelle le composé contient deux motifs de structure de formule générale (I), l étant = m = 1 et n = 0, A représentant un groupe méthyle, B COO, R¹ C₂H₄, R² un atome d'hydrogène, R⁴ un groupe méthyle, R⁵ un atome d'hydrogène et X et Y un atome d'oxygène et R³ le substituant pontant reliant les deux motifs de structure

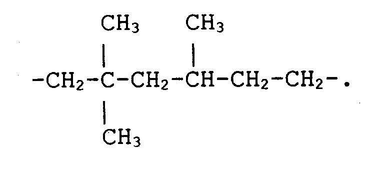

36. Utilisation d'un composé polymérisable selon la revendication 31, dans laquelle le composé contient deux motifs de structure de formule générale (I), l étant = m = 1 et n = 0, A représentant un groupe méthyle, B COO, R¹ C₂H₄, R² un atome d'hydrogène, R⁴ un groupe méthyle, R⁵ un atome d'hydrogène et X et Y un atome d'oxygène et R³ le substituant pontant reliant les deux motifs de structure 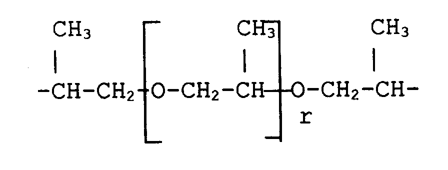 dans lequel r ≥ 1.

37. Utilisation d'un composé polymérisable selon la revendication 36, dans laquelle r = 5.

38. Utilisation d'un composé polymérisable selon une des revendications 24 à 37 , dans laquelle le composé polymérisable est un monomère.

39. Utilisation d'un composé polymérisable selon la revendication 31, dans laquelle le composé polymérisable est un prépolymère.

40. Utilisation d'un composé polymérisable selon une des revendications 24 à 39, dans laquelle le matériau dentaire est un homo- ou un co-polymère.

41. Utilisation d'un composé polymérisable selon une des revendications 24 à 39, dans laquelle le matériau dentaire est un matériau composite.

42. Utilisation d'un composé polymérisable selon une des revendications 24 à 39, dans laquelle le matériau dentaire est un matériau de moulage, un matériau de remplissage, un matériau de remplissage temporaire, un matériau de sous-remplissage ou un matériau de doublure.

43. Utilisation d'un composé polymérisable selon une des revendications 24 à 42, dans laquelle le composé est polymérisé par rayonnement.

44. Utilisation du composé selon une des revendications 8 à 12, et 14 comme agent de réticulation, dans laquelle G est un atome d'hydrogène et les motifs de structure de formule générale (I) sont reliés entre eux via un substituant pontant R².
